# FASCICULE DE BREVET EUROPEEN

(11) **EP 0 692 241 B1**
(45) Date de publication et mention de la délivrance du brevet: **21.08.1996**
(21) Numéro de dépôt: 95401439.5
(22) Date de dépôt: 19.06.1995
(51) Int. Cl.: A61K 7/00

(54) **Composition cosmétique et/ou dermatologique gélifiée, riche en solvant et contenant des particules creuses**
Gelartige kosmetische und/oder dermatologische Zusammensetzung enthaltend hohle Teilchen und eine grosse Menge an Lösungsmittel
Gelified cosmetic and/or dermatological composition containing hollow particles and high amounts of solvent

(30) Priorité: 11.07.1994 FR 9408568
(43) Date de publication de la demande: 17.01.1996
(73) Titulaire: L'OREAL, F-75008 Paris (FR)
(72) Inventeur: Sebillotte-Arnaud, Laurence, F-94000 Creteil (FR)
(74) Mandataire: Lhoste, Catherine

(56) Documents cités:
- EP-A- 0 614 656

## Description

L'invention se rapporte à une composition cosmétique et/ou dermatologique riche en solvant organique et contenant des particules creuses. Cette composition se présente sous forme d'un gel blanc ou coloré destiné au soin et/ou au traitement spécifique de la peau du visage, du corps humain y compris le cuir chevelu.

L'invention se rapporte aussi à une utilisation de cette composition pour le traitement cosmétique de la peau et en particulier pour prévenir et/ou lutter contre la pigmentation de la peau, contre la peau grasse et/ou les surcharges pondérales.

Elle se rapporte aussi à l'utilisation de cette composition pour la préparation d'une pommade ou d'un onguent destiné au traitement dermatologique de la peau ainsi qu' à un procédé de traitement cosmétique de la peau.

Les compositions classiquement utilisées dans les domaines cosmétique et/ou dermatologique sont des émulsions eau-dans-huile (E/H), des émulsions huile-dans-eau (H/E), ou des gels aqueux, dans lesquels il est souvent difficile, voire même impossible d'incorporer certains actifs comme l'acide kojique, l'acide caféique, l'acide salicylique et ses dérivés, les filtres solaires, les agents amincissants, les agents anti-radicaux libres, les vitamines, les répulsifs pour insectes, utilisés dans différents types de traitement comme la lutte contre le vieillissement, I'acné, les surcharges pondérales ou pour favoriser la circulation sanguine, etc.

En général, ces actifs ont tendance à recristalliser ou à se dégrader. Il s'ensuit une perte d'efficacité plus ou moins importante de ces compositions, selon le degré de recristallisation et/ou de dégradation, ce qui va à l'encontre de l'objectif recherché. En outre, cette recristallisation ou dégradation peut modifier la stabilité globale de ces compositions ainsi que leur aspect, ce qui peut détourner l'utilisateur de ces compositions à traitement spécifique. Par ailleurs, leur dissolution dans une composition quelconque nécessite souvent de chauffer cette dernière, ce qui est relativement gênant pour des actifs sensibles à la chaleur comme les vitamines, certains extraits de plante ou certaines protéines.

Pour que ces émulsions E/H ou H/E soient stables (non séparation des phases aqueuse et huileuse), il est nécessaire d'utiliser des émulsionnants (ou tensioactifs). Malheureusement, ces tensioactifs sont souvent irritants pour la peau. En outre, ces émulsions manquent souvent de fraîcheur à l'application, ce qui peut gêner leurs utilisations pendant les périodes chaudes de l'année et/ou dans les pays chauds. Un gel aqueux est beaucoup plus apprécié dans ces conditions d'utilisation, mais sa trop grande quantité d'eau ne permet pas d'y introduire ces actifs.

Il subsiste donc le besoin d'une composition stable, ayant tous les avantages d'un gel, utilisable dans les domaines cosmétique et/ou dermatologique, permettant une solubilisation suffisante des actifs généralement utilisés dans ces domaines en vue d'une efficacité maximum.

Ainsi, l'invention a pour objet une composition cosmétique et/ou dermatologique contenant un milieu hydrophile et au moins un agent gélifiant, caractérisée en ce qu'elle comprend des particules creuses et en ce que le milieu contient au moins 20 % en poids de solvant organique, par rapport au poids total de la composition, et moins de 50% en poids d'eau par rapport au poids total de la composition.

La composition de l'invention a une certaine consistance et/ou tenue ; elle n'est pas filante, c'est-à-dire qu'elle ne forme pas de fil lorsqu'on la prend au doigt. Elle se présente en outre sous forme d'une crème blanche ou colorée (en présence de colorants), onctueuse et brillante, aspect très recherché par les utilisateurs.

L'utilisation d'une composition gélifiée permet l'emploi d'une grande quantité de solvant, ce qui est difficilement réalisable avec une émulsion E/H ou H/E : l'introduction de solvant dans une émulsion casse cette dernière.

La composition de l'invention présente, en outre, l'avantage d'apporter une très grande douceur et un grand confort à l'application. Ainsi, la présence des particules permet, entre autre, d'introduire des corps gras en grande quantité dans la composition, sans ressentir aucune sensation de gras et/ou de toucher collant.

Cette composition, contrairement aux émulsions, ne contient peu ou pas de tensioactifs. Par ailleurs, contrairement aux processus d'obtention classiques des compositions gélifiées ou émulsionnées, il n'est pas nécessaire de chauffer pour former la composition. Ceci facilite donc sa fabrication et permet d'utiliser des actifs sensibles à la chaleur comme les vitamines ou les extraits de végétaux. Il s'ensuit donc une efficacité améliorée par rapport aux compositions obtenues par chauffage.

Cette composition est particulièrement bien adaptée à la solubilisation d'actifs utilisés dans les domaines cosmétique et dermatologique. Aussi, la composition de l'invention contient, en outre, au moins un actif choisi notamment parmi les actifs dépigmentants, kératolytiques, amincissants, de traitement de l'acné, anti-inflammatoires, les actifs destinés au traitement de la peau grasse.

Comme actifs solubilisables dans la composition, on peut citer les dépigmentants comme l'acide caféique, l'hydroquinone les actifs contre la peau grasse comme l'acide rétinoïque et ses dérivés, le péroxyde de benzoyle, l'octopirox ou des kératolytiques comme l'acide salicylique et ses dérivés comme le n-octanoyl-5-salicylique les antiinflammatoires comme le 17 valérate de β-méthasone, l'hydrocortisone, l'acide β-glycyrrhétinique ; les antibiotiques comme l'érythromycine et ses dérivés, la clindamycine et ses dérivés les antifongiques comme le miconazole, l'éconazole ; les amincissants comme la caféine, le centella asiatica ou l'acide asiatique et ses dérivés les actifs stimulant la repousse des cheveux comme le minoxidil les actifs favorisant la microcirculation comme le ruscus les actifs rafraîchissants comme le menthol et le camphre.

Le milieu hydrophile peut contenir de préférence moins de 30 % d'eau par rapport au poids total de la composition. De plus, le pH de ce milieu hydrophile peut varier de 3 à 12 en fonction du gélifiant et/ou des actifs utilisés.

Il est possible d'obtenir une composition dont l'aspect macroscopique reste homogène et dans laquelle le ou les actifs ne recristallisent pas et/ou ne se dégradent pas, notamment pendant au moins 2 mois à 45 °C.

Le ou les gélifiants sont choisis parmi les polymères naturels, synthétiques et les émulsions de polymères.

Ce sont des polymères ou copolymères d'acides organiques carboxyliques insaturés ou d'esters insaturés, les dérivés polysaccharidiques, les gommes, les polyvinylpyrrolidones et leurs dérivés.

Les agents gélifiants polymériques sont par exemple : les polymères ou copolymères carboxyvinyliques vendus sous la dénomination Carbopol par la Société Goodrich, les copolymères acide acrylique/méthacrylate de stéaryle, le polyglycérylméthacrylate vendu sous la dénomination Lubrajel par la Société Guardian, le polyglycérylacrylate vendu sous la dénomination Hispagel par la Société Hispano Chimica.

Les agents gélifiants se présentant sous forme d'émulsions E/H sont par exemple:
- les copolymères anioniques dont la formule générale est décrite dans la demande de brevet EP-A-0503 853.
- les copolymères cationiques dont la formule générale est décrite dans le document EP-0395 282 de la Société Allied Colloids Limited. Ces polymères sont par exemple vendus sous la dénomination Salcare SC 90, Salcare SC 91, Salcare SC 92, Salcare SC 95. On peut également utiliser ceux vendus sous la dénomination Bozepol C Nouveau, PAS 5194, PAS 5193 par la Société Hoechst.

Ces gélifiants sont utilisés dans les quantités habituellement employées pour obtenir un gel. Par exemple on utilise, en matière active, de 0,1 % à 10 % en poids de gélifiant, par rapport au poids total de la composition, et mieux de 1 % à 8 % et encore mieux de 3 % à 6 %.

Le ou les solvants utilisables dans l'invention sont des solvants hydrophiles associés éventuellement à des solvants lipophiles ou à la fois hydrophiles et lipophiles. En particulier, ces solvants sont des solvants cosmétiquement et/ou dermatologiquement acceptables (tolérance, toxicologie et toucher acceptables).

Les solvants hydrophiles peuvent représenter de 5 % à 90 % du poids total de la composition et de préférence de 20 % à 70 % et mieux de 50 % à 70 %. Ces solvants hydrophiles sont par exemple des mono-alcools inférieurs linéaires ou ramifiés ayant de 1 à 8 atomes de carbone comme l'éthanol, le propanol, le butanol, l'isopropanol, l'isobutanol ; des polyéthylène glycols ayant de 6 oxydes d'éthylène à 80 oxydes d'éthylène ; des polyols tels que le propylène glycol, l'isoprène glycol, le butylène glycol, le glycérol, le sorbitol les mono-ou di-alkyle d'isosorbide dont les groupements alkyle ont de 1 à 5 atomes de carbone comme le diméthyl isosorbide les éthers de glycol comme le diéthylène glycol monométhyl ou mono-éthyl éther et les éthers de propylène glycol comme le dipropylène glycol méthyl éther.

De préférence, la composition de l'invention contient au moins 10 % en poids, par rapport au poids total de la composition, d'alcools inférieurs.

Comme solvants à la fois lipophiles et hydrophiles, on peut citer des polyols tels que l'isoprène glycol ; des dérivés de polypropylène glycol (PPG) tels que les esters de polypropylène glycol et d'acide gras, de PPG et d'alcool gras comme le PPG-23 oléyl éther et le PPG-36 oléate. Ces solvants sont utilisés en combinaison avec les solvants hydrophiles avec une concentration allant de 0 % à 50 % du poids total de la composition et de préférence de 0 % à 30 % et mieux de 0 % à 10 %.

On peut éventuellement utiliser des solvants lipophiles associés aux solvants hydrophiles et/ou à la fois hydrophiles et lipophiles. Ces solvants lipophiles représentent de 0 % à 50 % du poids total de la composition et de préférence de 0 % à 30 % et mieux de 0 % à 10 %. Ces solvants sont en particulier des esters gras tels que l'adipate de diisopropyle, l'adipate de dioctyle, le malate de dioctyle, l'isostéarate de glycéryle, l'acétostéarate d'octyle, l'octanoate de cétyle, le sébacate de diisopropyle des alcools de Guerbet (ou 2-alkyl-1-alcanols) de structure générale R₁-CHR₂-CH₂OH avec R₁ et R₂ identiques ou différents représentant un atome d'hydrogène ou un radical alkyle ayant de 1 à 10 atomes de carbone comme l'octyldodécanol, l'hexyldécanol, le cyclododécanol.

Les particules de l'invention présentent, en particulier, une masse volumique choisie dans la gamme allant de 15 kg/m³ à 200 kg/m³ et mieux au plus égale à 120 kg/m³, par exemple de 40 kg/m³ à 100 kg/m³, et encore mieux de 60 kg/m³ à 80 kg/m³. Pour obtenir cette faible masse volumique, on utilise avantageusement des particules de polymères ou copolymères expansés de préférence à base d'acrylonitrile et d'un monomère acrylique ou styrénique et/ou de chlorure de vinylidène.

On peut par exemple utiliser un copolymère contenant: de 0 % à 60 % de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100. Le monomère acrylique est par exemple un acrylate ou méthacrylate de méthyle ou d'éthyle. Le monomère styrénique est par exemple l'α-méthyl-styrène ou le styrène.

De préférence, les particules utilisées dans la présente invention sont des particules creuses d'un copolymère expansé de chlorure de vinylidène et d'acrylonitrile ou de chlorure de vinylidène, d'acrylonitrile et de méthacrylate. Ces particules peuvent être sèches ou hydratées.

Les particules de l'invention peuvent être obtenues, par exemple, selon les procédés des brevets et demandes de brevet EP-56219, EP-348372, EP-486080, EP-320473, EP-112807 et US-3615972.

La cavité interne des particules contient en principe un gaz qui peut être de l'air, de l'azote ou un hydrocarbure comme l'isobutane ou l'isopentane.

De façon avantageuse, les particules de l'invention ont une granulométrie allant de 5 µm à 200 µm, voire de 1 µm à 100 µm et encore mieux allant de 10 µm à 50 µm, et encore mieux de 10 µm à 30 µm.

Les particules utilisables dans l'invention sont par exemple les microsphères de terpolymère expansé de chlorure de vinylidène, d'acrylonitrile et de méthacrylate, vendues sous la marque EXPANCEL par la société Nobel Casco sous les références 551 DE 50 (granulométrie d'environ 40 µm), 551 DE 20 (granulométrie d'environ 30 µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23, ou ayant une granulométrie d'environ 34 µm et une masse volumique d'environ 20 kg/m³, appelées ci-dessous EL 43.

Ces particules confèrent en outre aux émulsions les contenant de la légèreté et de la matité à l'application.

Dans les compositions de l'invention, on utilise de préférence de 0,1 % à 10 % en poids de particules dans la composition, par rapport au poids total de la composition, et mieux de 0,5 % à 5 % en poids.

Afin que la composition de l'invention soit plus agréable à utiliser (plus douce à l'application, plus nourrissante, plus émolliente), il est possible d'ajouter une ou plusieurs huiles solubles ou non dans le milieu hydrophile, c'est-à-dire dans l'eau ou le solvant.

Les huiles solubles dans l'eau ou dans le milieu hydroalcoolique représentent de 0 % à 50 % du poids total de la composition, de préférence de 0 % à 30 % et mieux de 1 % à 15 %.

Les huiles partiellement solubles dans le solvant, utilisables dans l'invention, sont par exemple les cyclométhicones (cyclohexa-, cyclopenta- ou cyclotétra-diméthylsiloxane), les polydiméthyl siloxanes volatils, linéaires (à 2, 3, ou 4 fonctions siloxane) ou encore les phényldiméthyl siloxanes.

Les huiles solubles dans l'eau, utilisables dans l'invention, sont par exemple les silicones hydrosolubles telles que les :
- polydiméthyl siloxanes oxyéthylénés à motifs amide comme par exemple ceux vendus sous le nom Silwax AX ou Silwax DCA-100 par la société Siltech ;
- polydiméthyl siloxanes oxyéthylénés à groupements stéarate comme par exemple ceux vendus sous le nom Silwax WD-IS par la société Siltech ;
- polydiméthyl siloxanes oxyéthylénés comme par exemple ceux vendus sous le nom Belsil DMC 6038 par la société Wacker;
- stéaroxy polydiméthyl siloxanes comme par exemple ceux vendus sous le nom Belsil SDM 6022 par la société Wacker.

On peut aussi utiliser des huiles insolubles dans le milieu hydroalcoolique. Ces huiles représentent de 0 % à 20 % en poids et de préférence de 1 % à 15 %. Ces huiles insolubles sont par exemples :
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah, l'huile de noisette, le beurre de karité et sa fraction liquide, l'huile de palme, l'huile de noyau d'abricot, l'huile de calophyllum, l'huile de son de riz, l'huile de germe de maïs, l'huile de germe de blé, l'huile de soja, l'huile de tournesol, l'huile d'onagre, l'huile de carthame, l'huile de passiflore et l'huile de seigle,
- les huiles synthétiques telles que l'huile de purcellin, les esters gras dont le myristate de butyle, le myristate d'isopropyle, le stéarate d'héxadécyle, le stéarate d'isopropyle, le stéarate d'octyle, le stéarate d'isocétyle, I'oléate de décyle, le laurate d'héxyle, le dicaprylate de propylène glycol, les octanoates de glycol ou de glycérol, les ricinoléates d'alcools mono- ou polyfonctionnels (de cétyle notamment), les triglycérides d'acides gras notamment d'acides caprylique/caprique ou d'acides gras saturés en C₁₀ à C₁₈, les esters dérivés d'acide lanolique tels que le lanolate d'isopropyle, le lanolate d'isocétyle, les isoparaffines et les huiles fluorées et perfluorées.

Les huiles utilisées peuvent également contenir un ou plusieurs actifs cosmétiques et/ou thérapeutiques lipophiles, notamment ceux utilisés de manière habituelle dans la fabrication et l'obtention des compositions cosmétiques et/ou pharmaceutiques. Ces actifs peuvent être, par exemple, des agents anti-radicaux libres des céramides des filtres solaires (notamment ultraviolets) tels que le paraméthoxycinnamate de 2-éthylhexyle et en particulier celui commercialisé sous la dénomination "Parsol MCX" par la Société GIVAUDAN ou la 2-hydroxy-4-méthoxybenzophénone et en particulier celle vendue sous la dénomination commerciale "Uvinul M40" par la Société BASF des répulsifs pour insectes tels que le cétyl-3 éthylaminopropionate de n-butyle ou le diéthylamide-N,N-caprylique; des agents amincissants tels que le nicotinate de D-L a-tocophérol, l'extrait huileux de ginseng (Panax Ginseng), l'extrait huileux de lierre grimpant (Hedera Helix), l'extrait huileux de fleurs sèches d'arnica (Arnica montana L) et l'extrait huileux d'algues (Fucus Vesiculosus). Il va de soi que la liste ci-dessus d'actifs lipophiles susceptibles d'être introduits dans la composition de l'invention n'est nullement exhaustive.

Les huiles peuvent également contenir, selon l'application envisagée, un ou plusieurs additifs de formulation lipophiles tels que des agents conservateurs, antioxydants ou émollients, des huiles parfumées voire même des parfums.

On peut trouver d'autres types d'additifs dans la composition comme les dérivés de cellulose tels que la carboxyméthylcellulose, l'hydroxyméthylcellulose, les hydroxyéthylcelluloses, vendues sous la dénomination Idroramnosan, Liporamnosan, Alcoramnosan par la Société Vevy Europe, l'hydroxypropylcellulose, vendue sous la dénomination Veegum, etc., ainsi que des additifs hydrophiles comme les hydratants du type polyols (glycérine, sorbitol). Les dérivés peuvent être utilisés de 0 % à 5 % du poids total de la composition et mieux de 0,1 % à 3 % voire de 0,5 % à 2 % et les polyols peuvent être utilisés jusq'à 10 % et notamment de 2 % à 6 %.

L'invention se rapporte aussi à un procédé de traitement cosmétique, par voie topique, de la peau, qui consiste à appliquer sur la peau, du visage, du cuir chevelu et/ou du corps humain une composition telle que définie précédemment. Le type de traitement est fonction du ou des actifs solubilisés dans la composition.

Plus spécialement, l'invention se rapporte à un procédé pour prévenir et/ou lutter contre la pigmentation de la peau, contre la peau grasse et/ou les surcharges pondérales, consistant à appliquer sur la peau une composition telle que définie ci-dessus contenant au moins un actif dépigmentant ou antipigmentant, au moins un actif amincissant ou au moins un actif pour traiter la peau grasse.

L'invention a encore pour objet l'utilisation de la composition ci-dessus pour traiter la peau du visage et/ou du corps humain et notamment pour prévenir et/ou lutter contre la pigmentation de la peau et/ou contre la peau grasse du visage, du cuir chevelu et/ou du corps humain et pour prévenir et/ou lutter contre les surcharges pondérales.

L'invention a encore pour objet l'utilisation de la composition ci-dessus pour préparer une pommade ou un onguent destiné à traiter thérapeutiquement le visage et/ou le corps humain.

La description qui suit est donnée à titre illustratif et non limitatif. Les concentrations des différents constituants entrant dans les compositions exemplifiées ci-après, sont données en pourcentage pondéral. Ces différentes compositions ont été obtenues selon le mode opératoire suivant.

On solubilise le ou les actifs dans le solvant ou le mélange de solvants choisi (phase A), puis on gélifie avec le copolymère (phase C) par simple homogénéisation à température ambiante au moyen d'une turbine à hélice. Les microsphères creuses expancées sont empâtées avec les huiles émollientes (phase B) lorsque les huiles sont présentes. Le mélange est ensuite incorporé, toujours à température ambiante, avec le même moyen. Dans le cas où il n'y a pas d'huile émolliente, les microsphères creuses expancées sont saupoudrées en final et incorporées au moyen d'une paie. Les formules sont soit fluides et non filantes, soit avec un seuil d'écoulement tel que le produit ne s'écoule pas sous son propre poids. Elles ont l'aspect d'une crème homogène, lisse, brillante, onctueuse et souple.

Dans les exemples qui suivent les quantités sont données en pourcentages pondérals.

### EXEMPLE 1 : CREME DEPIGMENTANTE

| ***Phase A*** | |
|---|---|
| Eau déminéralisée | qsp 100 % |
| Alcool éthylique à 96 °C dans de l'eau | 30 % |
| Polyéthylène glycol 8 fois oxyéthyléné | 30 % |
| Acide caféique | 1,2 % |
| Acide salicylique | 5 % |

| ***Phase B*** | |
|---|---|
| EXPANCEL EL 23 | 0,5 % |
| Fraction liquide de beurre de karité | 2 % |

| ***Phase C*** | |
|---|---|
| Gélifiant * | 5 % |

| ***Phase D*** | |
|---|---|
| Colorant, parfum | qs |
| Le produit obtenu a l'aspect d'une crème jaune, opaque, lisse, brillante et stable. Son pH est de 3. | |

| | |
|---|---|
| (*) Ce gélifiant correspond, pour les exemples 1 et 3, au produit tel que préparé dans l'exemple 1 de la demande de brevet EP-A-0503 853 déjà citée, c'est-à-dire un produit se présentant sous la forme d'une émulsion de type eau-dans-huile et contenant 40 % en poids environ d'un copolymère réticulé acrylamide (60 % en mole)/AMPS sodé (40 % en mole)/Méthylène bis-acrylamide (0,22 millimole/mole), et en poids environ d'un alcool gras éthoxylé ayant une valeur de la balance hydrophile/lipophile (HLB). | |

### EXEMPLE 2 : CREME DEPIGMENTANTE

| ***Phase A*** | |
|---|---|
| Eau déminéralisée | qsp 100 % |
| Alcool éthylique à 96 °C dans de l'eau | 30 % |
| Polyéthylène glycol 8 fois oxyéthyléné | 30 % |
| Acide caféique | 1,2 % |
| Acide n-octanoyl-5-salicylique | 2 % |

| ***Phase B*** | |
|---|---|
| EXPANCEL EL 23 | 0,5 % |
| Fraction liquide de beurre de karité | 2 % |

| ***Phase C*** | |
|---|---|
| SALCARE 95 Gélifiant | 6 % |

| ***Phase D*** | |
|---|---|
| Colorant, parfum | qs |
| Le produit obtenu a l'aspect d'une crème jaune, opaque, lisse, brillante et stable. Son pH est de 3. | |

### EXEMPLE 3 : CREME AMINCISSANTE

| ***Phase A*** | |
|---|---|
| Eau déminéralisée | qsp 100 % |
| Alcool éthylique à 96 °C dans de l'eau | 30 % |
| Caféine | 2 % |

| ***Phase B*** | |
|---|---|
| EXPANCEL EL 23 | 1 % |
| Extrait huileux de lierre grimpant | 5 % |

| ***Phase C*** | |
|---|---|
| Gélifiant * | 2 % |
| Lubrajel ^{(R)} par la Société Guardian | 20 % |
| Le produit obtenu a l'aspect d'une crème blanche, lisse, brillante et stable. Son pH est de 6. | |

| | |
|---|---|
| (*) Ce gélifiant correspond, pour les exemples 1 et 3, au produit tel que préparé dans l'exemple 1 de la demande de brevet EP-A-0503 853 déjà citée, c'est-à-dire un produit se présentant sous la forme d'une émulsion de type eau-dans-huile et contenant 40 % en poids environ d'un copolymère réticulé acrylamide (60 % en mole)/AMPS sodé (40 % en mole)/Méthylène bis-acrylamide (0,22 millimole/mole), et en poids environ d'un alcool gras éthoxylé ayant une valeur de la balance hydrophile/lipophile (HLB). | |

### EXEMPLE 4 : CREME PEAUX GRASSES

| ***Phase A*** | |
|---|---|
| Eau déminéralisée | qsp 100 % |
| Alcool éthylique à 96 °C dans de l'eau | 30 % |
| Polyéthylène glycol 8 fois oxyéthyléné | 30 % |
| Octopirox | 0,5 % |

| ***Phase B*** | |
|---|---|
| 551 DE 20 | 1 % |
| Cyclohexa diméthylsiloxane | 5 % |

| ***Phase C*** | |
|---|---|
| Carbopol 980 | 0,5 % |
| Eau | 20 % |

| ***Phase D*** | |
|---|---|
| Triéthanolamine | qsp pH = 7 |
| Le produit obtenu a l'aspect d'une crème brillante, onctueuse, douce. | |

## Revendications

1. Composition cosmétique et/ou dermatologique contenant un milieu hydrophile et au moins un agent gélifiant, caractérisée en ce qu'elle comprend des particules creuses et en ce que le milieu contient au moins 20 % en poids de solvant organique, par rapport au poids total de la composition, et moins de 50% en poids d'eau par rapport au poids total de la composition.

2. Composition selon la revendication 1, caractérisée en ce que les particules ont une masse volumique au plus égale à 120 kg/m³.

3. Composition selon l'une des revendications précédentes, caractérisée en ce que les particules ont une masse volumique allant de 40 kg/m³ à 100 kg/m³.

4. Composition selon l'une des revendications précédentes, caractérisée en ce que les particules ont une masse volumique allant de 60 kg/m³ à 80 kg/m³.

5. Composition selon l'une des revendications précédentes, caractérisée en ce que les particules ont une granulométrie allant de 5 µm à 200 µm.

6. Composition selon l'une des revendications précédentes, caractérisée en ce que les particules ont une granulométrie allant de 10 µm à 100 µm.

7. Composition selon l'une des revendications précédentes, caractérisée en ce que les particules ont une granulométrie allant de 10 µm à 50 µm.

8. Composition selon l'une des revendications précédentes, caractérisée en ce que les particules sont formées d'un polymère ou copolymère expansé de méthyl (méth)acrylate et/ou d'acrylonitrile et/ou de chlorure de vinylidène.

9. Composition selon l'une des revendications précédentes, caractérisée en ce que les particules représentent de 0,01 % à 10 % du poids total de la composition.

10. Composition selon l'une des revendications précédentes, caractérisée en ce que le milieu hydrophile contient moins de 30 % en poids d'eau par rapport au poids total de la composition.

11. Composition selon l'une des revendications précédentes, caractérisée en ce que le milieu hydrophile contient au moins un solvant organique choisi parmi les alcools mono- ou polyfonctionnels, les polyéthylène glycols éventuellement oxyéthylénés, les esters de propylène glycol, le sorbitol et ses dérivés, les dialkyles d'isosorbide, les éthers de glycol et les éthers de propylène glycol.

12. Composition selon l'une quelconque des revendications précédentes, caractérisée en ce que le solvant représente de 5 % à 90 % du poids total de la composition.

13. Composition selon l'une quelconque des revendications précédentes, caractérisée en ce que le gélifiant représente, (en matière active) de 0,1 % à 10 % du poids total de la composition.

14. Composition selon l'une quelconque des revendications précédentes, caractérisée en ce qu'elle contient au moins une huile soluble ou non dans le milieu hydrophile.

15. Composition selon la revendication 14, caractérisée en ce que l'huile représente de 0 % à 50 % du poids total de la composition.

16. Composition selon l'une quelconque des revendications précédentes, caractérisée en ce qu'elle contient au moins un actif cosmétiquement et/ou dermatologiquement acceptable.

17. Composition selon l'une quelconque des revendications précédentes, caractérisée en ce qu'elle contient au moins un actif dépigmentant, amincissant, ou pour traiter les peaux grasses.

18. Composition selon l'une quelconque des revendications précédentes, caractérisée en ce qu'elle contient, encore, au moins un additif choisi parmi les céramides, les agents conservateurs, les agents antioxydants, les agents émollients, les parfums.

19. Composition selon l'une quelconque des revendications précédentes, caractérisée en ce qu'elle contient au moins un actif choisi parmi l'acide caféique, l'acide salicylique et ses dérivés, la caféine, l'octopirox et leurs mélanges.

20. Utilisation de la composition selon l'une des revendications précédentes, pour traiter cosmétiquement la peau du visage, du corps et/ou du cuir chevelu.

21. Utilisation de la composition selon l'une des revendications précédentes, pour prévenir et/ou lutter cosmétiquement contre la pigmentation de la peau, contre la peau grasse et/ou les surcharges pondérales.

22. Procédé pour traiter cosmétiquement la peau du visage, du cuir chevelu et/ou du corps humain, consistant à appliquer sur la peau la composition selon l'une des revendications 1 à 19.

23. Utilisation de la composition selon l'une quelconque des revendications 1 à 19, pour préparer une pommade ou un onguent destiné à traiter thérapeutiquement le visage et/ou le corps humain.

## Claims

1. Cosmetic and/or dermatological composition containing a hydrophilic medium and at least one gelling agent, characterized in that it comprises hollow particles and in that the medium contains at least 20 % by weight of organic solvent, with respect to the total weight of the composition, and less than 50 % by weight of water with respect to the total weight of the composition.

2. Composition according to Claim 1, characterized in that the particles have a density not greater than 120 kg/m³.

3. Composition according to either of the preceding claims, characterized in that the particles have a density ranging from 40 kg/m³ to 100 kg/m³.

4. Composition according to one of the preceding claims, characterized in that the particles have a density ranging from 60 kg/m³ to 80 kg/m³.

5. Composition according to one of the preceding claims, characterized in that the particles have a particle size ranging from 5 µm to 200 µm.

6. Composition according to one of the preceding claims, characterized in that the particles have a particle size ranging from 10 µm to 100 µm.

7. Composition according to one of the preceding claims, characterized in that the particles have a particle size ranging from 10 µm to 50 µm.

8. Composition according to one of the preceding claims, characterized in that the particles are formed from an expanded polymer or copolymer of methyl (meth)acrylate and/or of acrylonitrile and/or of vinylidene chloride.

9. Composition according to one of the preceding claims, characterized in that the particles represent from 0.01 % to 10 % of the total weight of the composition.

10. Composition according to one of the preceding claims, characterized in that the hydrophilic medium contains less than 30 % by weight of water with respect to the total weight of the composition.

11. Composition according to one of the preceding claims, characterized in that the hydrophilic medium contains at least one organic solvent chosen from mono- or polyfunctional alcohols, optionally oxyethylenated polyethylene glycols, propylene glycol esters, sorbitol and its derivatives, dialkyl derivatives of isosorbide, glycol ethers and propylene glycol ethers.

12. Composition according to any one of the preceding claims, characterized in that the solvent represents from 5 % to 90 % of the total weight of the composition.

13. Composition according to any one of the preceding claims, characterized in that the gelling agent represents (as active material) from 0.1 % to 10 % of the total weight of the composition.

14. Composition according to any one of the preceding claims, characterized in that it contains at least one oil which is soluble or insoluble in the hydrophilic medium.

15. Composition according to Claim 14, characterized in that the oil represents from 0 % to 50 % of the total weight of the composition.

16. Composition according to any one of the preceding claims, characterized in that it contains at least one cosmetically and/or dermatologically acceptable active agent.

17. Composition according to any one of the preceding claims, characterized in that it contains at least one depigmenting or slimming active agent or active agent for treating greasy skins.

18. Composition according to any one of the preceding claims, characterized in that it also contains at least one additive chosen from ceramides, preserving agents, antioxidizing agents, emollients or fragrances.

19. Composition according to any one of the preceding claims, characterized in that it contains at least one active agent chosen from caffeic acid, salicylic acid and its derivatives, caffeine, octopirox and their mixtures.

20. Use of the composition according to one of the preceding claims for cosmetically treating the skin of the face, of the body and/or of the scalp.

21. Use of the composition according to one of the preceding claims for preventing and/or cosmetically controlling pigmentation of the skin or for cosmetically controlling greasy skin and/or excessive weight.

22. Process for cosmetically treating the skin of the face, of the scalp and/or of the human body which consists in applying, to the skin, the composition according to one of Claims 1 to 19.

23. Use of the composition according to any one of Claims 1 to 19 for preparing a salve or an ointment intended for the therapeutic treatment of the face and/or of the human body.

## Patentansprüche

1. Kosmetische und/oder dermatologische Zusammensetzung, die ein hydrophiles Medium und mindestens einen Gelbildner enthält, dadurch gekennzeichnet, daß sie Hohlpartikel enthält und dadurch, daß das Medium mindestens 20 Gew. -% organisches Lösungsmittel, bezogen auf das Gesamtgewicht der Zusammensetzung, und weniger als 50 Gew. -% Wasser, bezogen auf das Gesamtgewicht der Zusammensetzung, enthält.

2. Zusammensetzung nach Anspruch 1, dadurch gekennzeichnet, daß die Partikel eine Dichte von höchstens 120 kg/m³ aufweisen.

3. Zusammensetzung nach einem der vorhergehenden Ansprüche, dadurch gekennzeichnet, daß die Partikel eine Dichte von 40 bis 100 kg/m³ aufweisen.

4. Zusammensetzung nach einem der vorhergehenden Ansprüche, dadurch gekennzeichnet, daß die Partikel eine Dichte von 60 bis 80 kg/m³ aufweisen.

5. Zusammensetzung nach einem der vorhergehenden Ansprüche, dadurch gekennzeichnet, daß die Partikel eine Teilchengröße von 5 bis 200 µm aufweisen.

6. Zusammensetzung nach einem der vorhergehenden Ansprüche, dadurch gekennzeichnet, daß die Partikel eine Teilchengröße von 10 bis 100 µm aufweisen.

7. Zusammensetzung nach einem der vorhergehenden Ansprüche, dadurch gekennzeichnet, daß die Partikel eine Teilchengröße von 10 bis 50 µm aufweisen.

8. Zusammensetzung nach einem der vorhergehenden Ansprüche, dadurch gekennzeichnet, daß die Partikel aus einem expandierten Polymer oder Copolymer von Methyl(meth)acrylat und/oder Acrylnitril und/oder Vinylidenchlorid gebildet sind.

9. Zusammensetzung nach einem der vorhergehenden Ansprüche, dadurch gekennzeichnet, daß die Partikel 0,01 bis 10 % des Gesamtgewichts der Zusammensetzung ausmachen.

10. Zusammensetzung nach einem der vorhergehenden Ansprüche, dadurch gekennzeichnet, daß das hydrophile Medium weniger als 30 Gew. -% Wasser, bezogen auf das Gesamtgewicht der Zusammensetzung, enthält.

11. Zusammensetzung nach einem der vorhergehenden Ansprüche, dadurch gekennzeichnet, daß das hydrophile Medium mindestens ein organisches Lösungsmittel enthält, das unter Alkoholen mit einer oder mehreren Hydroxygruppen, gegebenenfalls ethoxylierten Polyethylenglykolen, Propylenglykolestern, Sorbit und seinen Derivaten, Dialkylisosorbiden, Glykolethern und Propylenglykolethern ausgewählt ist.

12. Zusammensetzung nach einem der vorhergehenden Ansprüche, dadurch gekennzeichnet, daß das Lösungsmittel 5 bis 90 % des Gesamtgewichts der Zusammensetzung ausmacht.

13. Zusammensetzung nach einem der vorhergehenden Ansprüche, dadurch gekennzeichnet, daß der Gelbildner (als Wirkstoff) 0,1 bis 10 % des Gesamtgewichts der Zusammensetzung ausmacht.

14. Zusammensetzung nach einem der vorhergehenden Ansprüche, dadurch gekennzeichnet, daß sie mindestens ein Öl enthält, das in dem hydrophilen Medium löslich ist oder nicht.

15. Zusammensetzung nach Anspruch 14, dadurch gekennzeichnet, daß das Öl 0 bis 50 % des Gesamtgewichts der Zusammensetzung ausmacht.

16. Zusammensetzung nach einem der vorhergehenden Ansprüche, dadurch gekennzeichnet, daß sie mindestens einen kosmetisch und/oder dermatologisch akzeptablen Wirkstoff enthält.

17. Zusammensetzung nach einem der vorhergehenden Ansprüche, dadurch gekennzeichnet, daß sie mindestens einen depigmentierenden Wirkstoff, schlanker machenden Wirkstoff oder Wirkstoff zur Behandlung von fettiger Haut enthält.

18. Zusammensetzung nach einem der vorhergehenden Ansprüche, dadurch gekennzeichnet, daß sie ferner mindestens einen Wirkstoff enthält, der unter Ceramiden, Konservierungsmittel, Antioxidantien, Softenern und Parfums ausgewählt ist.

19. Zusammensetzung nach einem der vorhergehenden Ansprüche, dadurch gekennzeichnet, daß sie mindestens einen Wirkstoff enthält, der unter Kaffeesäure, Salicylsäure und ihren Derivaten, Coffein, Octopirox und deren Gemischen ausgewählt ist.

20. Verwendung der Zusammensetzung nach einem der vorhergehenden Ansprüche zur kosmetischen Behandlung der Haut des Gesichts, des Körpers und/oder der Kopfhaut.

21. Verwendung der Zusammensetzung nach einem der vorhergehenden Ansprüche zur kosmetischen Vorbeugung und/oder Bekämpfung der Pigmentierung der Haut, gegen fettige Haut und/oder Übergewicht.

22. Verfahren zur kosmetischen Behandlung der Haut des Gesichts, der Kopfhaut und/oder des menschlichen Körpers, das darin besteht, auf die Haut eine Zusammensetzung nach einem der Ansprüche 1 bis 19 aufzutragen.

23. Verwendung der Zusammensetzung nach einem der Ansprüche 1 bis 19 zur Herstellung einer medizinischen oder pharmazeutischen Salbe, die zur therapeutischen Behandlung des Gesichts und/oder des menschlichen Körpers bestimmt ist.
